# EUROPÄISCHE PATENTANMELDUNG

(11) **EP 3 443 941 A1**
(43) Veröffentlichungstag der Anmeldung: **20.02.2019**
(21) Anmeldenummer: 18188939.5
(22) Anmeldetag: 14.08.2018
(51) Int. Cl.: A61F 5/455, A61F 5/44

(54) **VERSORGUNGSVORRICHTUNG FÜR INKONTINENZ BEI FRAUEN**

(30) Priorität: 18.08.2017 DE 202017104969 U
(71) Anmelder: Jovanov, Mile, 87463 Dietmannsried (DE)
(72) Erfinder: JOVANOV, Mile, 87463 Dietmannsried (DE)
(74) Vertreter: Pfister & Pfister Patent- und Rechtsanwälte

(57) **Zusammenfassung**

Die Erfindung betrifft eine Versorgungsvorrichtung für Inkontinenz bei Frauen umfassend ein Urinsammelelement mit einer Urinableitung, eine Unterhose mit einer Durchführung für die Urinableitung und ein Fixierelement, wobei die Urinableitung des Urinsammelelementes durch die Durchführung in der Unterhose geführt ist und das Fixierelement auf der, dem Urinsammelelement abgewandten Seite der Unterhose an der Urinableitung befestigt ist.

## Beschreibung

Die Erfindung betrifft eine Versorgungsvorrichtung für Inkontinenz bei Frauen umfassend ein Urinsammelelement mit einer Urinableitung, eine Unterhose mit einer Durchführung für die Urinableitung und ein Fixierelement. Die Urinableitung des Urinsammelelementes ist durch die Durchführung in der Unterhose geführt und das Fixierelement ist auf der, dem Urinsammelelement abgewandten Seite der Unterhose an der Urinableitung befestigt.

Um Abhilfe bei Inkontinenz bei Frauen zu schaffen, gibt es mehrere Lösungsansätze. Nach einem Lösungsansatz wird der ungewollt austretende Urin durch saugfähiges Material aufgenommen und gespeichert. Dies entspricht vom Grundgedanken der Verwendung einer Windel bei Kleinkindern. Nachteilig an dieser Lösung ist, dass sich das den Urin aufnehmende, saugfähige Material im vollgesaugten Zustand für die Trägerin unangenehm anfühlt. Zudem entstehen oft unangenehme Gerüche. Darüber hinaus wird bei schwerer Inkontinenz ein häufiger Wechsel der Einlagen erforderlich, was wiederum zu einem sehr hohen Müllaufkommen führt. Ein alternativer Lösungsansatz besteht darin, den austretenden Urin mit einer Aufnahmevorrichtung aufzufangen und in einen Speicherbehälter, beispielsweise einen Beutel, weiterzuleiten. Problematisch bei derartigen Aufnahmevorrichtungen ist die Abdichtung dieser Aufnahmevorrichtungen zum Körper. Zum einen ist die Anatomie bei jeder Frau unterschiedlich, was eine Aufnahmevorrichtungen mit unveränderbaren Außenabmessungen sinnlos macht. Des Weiteren verändert sich die Form des Körpers bei Bewegungen der Trägerin. So sind beispielsweise Aufnahmevorrichtungen die im Stehen der Patientin gut abdichten in einem liegenden Zustand der Patientin oft unbrauchbar, da sie dann nicht mehr abdichten und der Urin nicht in die Aufnahmevorrichtungen sondern daneben in die Kleidung abfließt.

Es ist somit Aufgabe der Erfindung, eine Versorgungsvorrichtung für Inkontinenz bei Frauen vorzuschlagen, welche zuverlässig versehentlich austretenden Urin von der Patientin entfernt und gleichzeitig angenehm zu tragen ist.

Die Aufgabe der Erfindung wird gelöst durch eine Versorgungsvorrichtung für Inkontinenz bei Frauen umfassend
- ein Urinsammelelement mit einer Urinableitung,
- eine Unterhose mit einer Durchführung für die Urinableitung,
- und ein Fixierelement,
wobei die Urinableitung des Urinsammelelementes durch die Durchführung in der Unterhose geführt ist und das Fixierelement auf der, dem Urinsammelelement abgewandten Seite der Unterhose an der Urinableitung befestigt ist und das Urinsammelelement an der Unterhose ortsfest und verdrehsicher festhält. Eine erfindungsgemäße Versorgungsvorrichtung umfasst zunächst ein Urinsammelelement welches aus der Harnröhre austretenden Urin aufnimmt und sammelt. Das Urinsammelelement fängt den Urin auf und leitet ihn von der Patientin weg. Das Urinsammelelement ist nicht saugfähig d.h. es nimmt den Urin nicht in sich auf. Am Urinsammelelement ist eine Urinableitung vorgesehen, durch welche der aufgefangene Urin abfließt. Die Urinableitung kann dabei beispielsweise als Rohrstutzen ausgeführt sein, auf den ein Abführschlauch aufgesteckt wird. Eine erfindungsgemäße Versorgungsvorrichtung umfasst weiterhin eine Unterhose, welche dazu beiträgt, dass das Urinsammelelement dauerhaft in der richtigen Position zur Patientin ausgerichtet ist. Beim Anbringen der Versorgungsvorrichtung wird die Unterhose nach dem Einbringen des Urinsammelelementes angezogen und hält dieses somit am Körper der Patientin. Die Unterhose weist eine Durchführung für die Urinableitung auf. Im einfachsten Fall wird diese Durchführung durch ein Loch in der Unterhose gebildet, welches sich bei angezogener Unterhose im Schritt, d.h. zwischen den Beinen der Patientin befindet. Die Urinableitung wird durch die Durchführung in der Unterhose gesteckt. Anschließend kann ein Abführschlauch zur Ableitung des aufgefangenen Urins auf die Urinableitung gesteckt werden. Eine erfindungsgemäße Versorgungsvorrichtung umfasst weiterhin ein Fixierelement, welches an der Urinableitung befestigt wird. Bei dem Fixierelement kann es sich im einfachsten Fall um einen Haltering handeln, welcher kraftschlüssig auf die Urinableitung aufgeschoben wird. Das Anlegen einer erfindungsgemäßen Vorrichtung erfolgt dabei in folgenden Schritten: zunächst wird das Urinsammelelement in der Scheide so platziert, dass aus der Harnröhre austretender Urin vom Urinsammelelement aufgenommen wird. Das Urinsammelelement weist Bereiche auf, mit denen es am Körper der Patientin befestigt werden kann. So kann das Urinsammelelement beispielsweise eine elastische formbare Schale aufweisen, welche zwischen den Schamlippen eingeklemmt wird. Zur Festlegung oder Befestigung des Urinsammelelementes am Körper werden jedoch noch weitere, weiter unten im Detail beschriebene Befestigungsbereiche vorgeschlagen. Jedes erfindungsgemäße Urinsammelelement weist aber zumindest Bereiche auf, mit denen es ohne weitere Hilfsmittel zunächst am Körper befestigt werden kann. Im nächsten Schritt beim Anlegen der Versorgungsvorrichtung wird eine spezielle Unterhose angezogen und die Urinableitung des bereits am Körper der Patienten platzierten Urinsammelelementes durch eine Durchführung in der Unterhose geschoben. Schließlich wird außerhalb der Unterhose das Fixierelement auf der Urinableitung befestigt. Die Unterhose ist somit zwischen Urinsammelelement und Fixierelement eingeklemmt. Andersherum betrachtet sind Urinsammelelement und Fixierelement an der Unterhose befestigt. Hier liegt nun ein großer Vorteil einer erfindungsgemäßen Versorgungsvorrichtung. Die Unterhose dient in spezieller Weise als Hilfe, das Urinsammelelement so am Körper der Patientin zu fixieren, so dass es auch bei Bewegungen der Patientin eine sichere Abdichtung zur Umgebung bereitstellt. Die Unterhose hält somit das Urinsammelelement, ergänzend zu den haltenden Bereichen, die das Urinsammelelement selbst aufweist, an der richtigen Position. Durch die Festlegung des Urinsammelelementes relativ zur Unterhose, wozu das Fixierelement eingesetzt wird, wird insbesondere auch ein Verdrehen des Urinsammelelementes relativ zum Körper der Patientin verhindert. Durch eine erfindungsgemäße Versorgungsvorrichtung wird eine Unterhose dazu genutzt, die Auflagefläche zur Ausrichtung und Fixierung des Urinsammelelementes zu vergrößern. Die Unterhose kann dabei spezielle Bereiche aufweisen, in denen die Haftung am Körper nochmals zusätzlich verbessert ist. Dies kann beispielsweise durch eine partielle Gummierung erfolgen. Es gibt viele verschiedene Unterhosen, die kostengünstig und schnell verfügbar sind. Somit ist eine Anpassung einer erfindungsgemäßen Versorgungsvorrichtung an die Anatomie jeder Trägerin sehr einfach möglich. Durch die Auswahl einer passenden Unterhose ist in Kombination mit den anderen Komponenten der Versorgungsvorrichtung eine komfortable, einfache und sichere Hilfe bei Inkontinenz bereitgestellt.

Des Weiteren ist in dem Vorschlag vorteilhafter Weise vorgesehen, dass das Fixierelement form- und/oder kraftschlüssig mit der Urinableitung verbunden ist und die Unterhose zwischen dem Urinsammelelement und dem Fixierelement eingeklemmt ist. In dieser Ausführungsform wird die Verbindung zwischen dem Fixierelement und der Urinableitung formschlüssig oder kraftschlüssig realisiert. Eine besonders einfache, kraftschlüssige Verbindung wird dadurch erzielt, dass der Außendurchmesser einer zylindrisch geformten Urinableitung etwas größer ausgelegt wird als der Innendurchmesser eines ringförmigen Fixierelementes aus einem elastischen Material. Beim Aufstecken des ringförmigen Fixierelementes auf die Urinableitung wird dieses aufgedehnt und durch die bei dieser Ausdehnung entstehenden elastischen Rückstellkraft befestigt. Eine mögliche, formschlüssige Ausführung dieser Verbindung ist dadurch möglich, auf dem Außenumfang der Urinableitung ein Gewinde vorzusehen, welches zu einem Innengewinde im Fixierelement passt. In dieser Ausführungsform kann dann das Fixierelement einfach auf die Urinableitung aufgeschraubt werden. Selbstverständlich gehören auch andere Ausführungsformen dieser Verbindung mit zur Erfindung. Auch eine Kombination aus Kraftschluss und Formschluss ist denkbar, ebenso wie eine stoffschlüssige Verbindung, wie beispielsweise eine Klebeverbindung.

Bei einer bevorzugten Ausgestaltung des Vorschlags ist vorgesehen, dass die Urinableitung einen Anschlussbereich für einen Abflussschlauch aufweist, welcher den Urin zu einem Sammelbeutel führt. In dieser Ausführungsform ist an der Urinableitung ein spezieller Anschlussbereich vorgesehen, der zur sicheren und dichten Befestigung eines Abflussschlauches für den Urin dient. Ein solcher Anschlussbereich kann beispielsweise Rippen aufweisen, die das Ende des ausgestreckten Abflussschlauches aufdehnen. Durch diese Aufdehnung wiederum ist eine sichere Abdichtung durch die elastische Rückstellkraft im Schlauchende auf dem Anschlussbereich sichergestellt. Der Anschlussbereich kann aber auch als Gewindestutzen ausgeführt sein, auf welchen ein ebenfalls mit einem Gewinde versehenes Schlauchende aufgeschraubt wird.

Des Weiteren ist vorgesehen, dass die Unterhose zumindest einen Haltebund aufweist, der aus einem elastischen Material gefertigt ist und fest am Körper der Trägerin anliegt. Wie bereits oben beschrieben wird bei einer erfindungsgemäßen Versorgungsvorrichtung die gesamte Fläche der Unterhose dazu verwendet, die Versorgungsvorrichtung sicher und stabil am Körper der Patientin zu fixieren. Diese Fixierung kann dadurch weiter verbessert werden, dass einer oder mehrere Haltebünde an der Unterhose vorgesehen sind die aus einem elastischen Material mit hoher Rückstellkraft gefertigt sind. Diese Haltebünde sorgen für ein verrutschfestes Anliegen der Unterhose am Körper. Günstigerweise können solche Haltebünde am oberen Rand der Unterhose oder an den Rändern der Löcher für die Durchführung der Beine angebracht werden. Die Bereiche zwischen den Haltebünden können dann aus dünneren, weniger elastischem Material gefertigt werden, welches wiederum atmungsaktiver als das Material der Haltebünde gestaltet werden kann. So lässt sich eine Unterhose gestalten, welche zum einen angenehm zum Tragen ist und zum anderen so stabil am Körper fixiert ist, dass auch bei Bewegung der Patientin kein Verrutschen erfolgt.

In einer vorteilhaften Ausgestaltung ist vorgesehen, dass der Haltebund rutschfest beschichtet ist. In dieser Ausführungsform ist der Haltebund mit einem Material beschichtet, welches sich besonders rutschfest auf der Haut der Trägerin verhält. Ein geeignetes Material für eine solche Beschichtung ist Beispielsweise eine Gummimischung, wie sie auch zur Fixierung von Hosen für Fahrradfahrer verwendet wird. Generell ist jedes Material geeignet, welches sich dauerhaft fest auf dem Haltebund aufbringen lässt und einen hohen Reibungskoeffizienten gegenüber Haut aufweist.

Geschickter Weise ist vorgesehen, dass die Durchführung in der Unterhose bereits bei der Herstellung der Unterhose vorgesehen wird. In dieser Ausführungsform wird die Durchführung in der Unterhose bereits bei deren Herstellung eingebracht. Dies hat den Vorteil, dass die Durchführung automatisiert und mit hoher Qualität eingebracht werden kann. Es ist beispielsweise auch möglich die Durchführung im Stoff zu umnähen oder deren Ränder zu beschichten, um ein Ausreißen oder eine Vergrößern der Durchführung zu verhindern.

In einer weiteren bevorzugten Ausführungsform ist vorgesehen, dass die Durchführung in der Unterhose passend zur individuellen Anatomie der Trägerin nachträglich in der Unterhose einbringbar ist. Alternativ zur Einbringung der Durchführung bei der Herstellung der Unterhose kann die Durchführung auch in einer individuellen Position bei der Anpassung der Versorgungsvorrichtung an die Patientin vorgenommen werden. Dies hat den Vorteil, dass die Anbringung der Durchführung in exakt der Position erfolgt, die optimal für eine Platzierung der Versorgungsvorrichtung am Körper ist. Für diese optimale Platzierung muss allerdings das Einbringen durch die Patientin selbst oder durch geschultes Hilfspersonal erfolgen.

Des Weiteren ist günstiger Weise vorgesehen, dass das Urinsammelelement zumindest eine Positionsführung aufweist und die Unterhose und/oder das Fixierelement eine Führungsaufnahme aufweist/aufweisen und die Positionsführung und die Führungsaufnahme formschlüssig miteinander in Eingriff bringbar sind um ein Verdrehen des Urinsammelelementes zur Unterhose und/oder zum Fixierelement zu verhindern. In dieser Ausführungsform ist eine Kombination aus den Elementen Positionsführung und Führungsaufnahme vorgesehen, welche die Verdrehsicherheit des Urinsammelelementes weiter verbessert. Auf der Seite des Urinsammelelementes ist dazu eine Positionsführung vorgesehen. Diese Positionsführung kann beispielsweise als eine Art Flügel ausgeführt sein, der benachbart zur Urinableitung angeordnet ist. Die Unterhose weist als Führungsaufnahme die Komplementärform dieser Positionsführung auf. Die Positionsführung passt somit wie ein Schlüssel in die Führungsaufnahme, welche in diesem Denkmodell das Schloss bildet. Durch diesen Formschluss zwischen den beiden Elementen wird ein Verdrehen des Urinsammelelementes gegenüber der Unterhose verhindert. Alternativ oder zusätzlich kann das Fixierelement ebenfalls eine komplementär zur Positionsführung ausgeführte Führungsaufnahme aufweisen.

Vorteilhafter Weise ist vorgesehen, dass das Urinsammelelement eine Auffangschale aufweist, welche schalenförmig und elastisch ausgebildet ist und die Auffangschale zwischen den Schamlippen der Trägerin einbringbar ist, wobei eine Auffangöffnung der Auffangschale nach oben orientiert ist und den Harnröhrenausgang der Trägerin umgibt. In dieser Ausführungsform weist das Urinsammelelement eine Auffangschale auf. Diese Auffassungsschale ist elastisch ausgeführt und weist einen Rand auf, welcher zwischen den Schamlippen in der Scheide aufliegt und den Harnröhrenausgang umgibt. Eine einfache Ausgestaltung der Auffangschale ist in der Form einer Halbkugel. Diese Halbkugel ist elastisch ausgeführt, sodass sie beim Einführen zwischen die Schamlippen eine ovale Grundform einnimmt. Der durch das Anliegen an den Schamlippen in eine ovale Form gedrückte obere Rand der auf Fangschale dichtet dann das Innere der auf Fangschale, und damit den Ausgang der Harnröhre, gegenüber der äußeren Umgebung ab. Aus dem Harnröhrenausgang austretender Urin wird von der Auffangsschale aufgenommen und weitergeleitet.

Des Weiteren ist in dem Vorschlag vorteilhafter Weise vorgesehen, dass das Urinsammelelement zusätzlich zur Auffangschale eine Fixiereinlage aufweist, welche lösbar fest mit der Auffangschale verbindbar ist, wobei die Fixiereinlage als gewölbte Platte ausgeführt ist und eine, in der Draufsicht betrachtet, im Wesentlichen dreieckige Form aufweist. In dieser Ausführungsform ist die Auffangschale lösbar fest mit einer Fixiereinlage verbunden. Diese Fixiereinlage dient der verbesserten Fixierung und Ausrichtung der Versorgungsvorrichtung. Die Fixiereinlage ist so geformt, dass sie zwischen die Beine der Patientin passt. Zwischen den Beinen verläuft die fixierte Einlage vom Anusbereich über den Bereich der Schamlippen bis hin zum vorderen Unterbauch im Bereich der Schambehaarung. Dadurch, dass die Fixiereinlage im Anusbereich schmal ausgeführt und im Bereich des vorderen Unterbauches breit ausgeführt ist erhält die Fixiereinlage Von oben betrachtet eine im wesentlichen dreieckige Form. Von der Seite betrachtet weist die Fixiereinlage eine gewölbte Form auf, die der Anatomie der Patientin vom Anusbereich zum vorderen Unterbauch hin folgt. Die Fixiereinlage ist so geformt, dass sie so verläuft, wie eine normal angezogene Unterhose im Bereich zwischen den Beinen verlaufen würde, nämlich optimal anliegend am Körper der Patientin. Die Fixiereinlage ist allerdings aus einem biegsamen aber ansonsten steifen Material geformt. Beim Anlegen der Versorgungsvorrichtung wird die Auffangschale mit der Fixiereinlage verbunden. Anschließend wird die Auffangschale Zwischen den Schamlippen so platziert, dass eine Abdichtung des Harnröhren Ausgangs gegeben ist. Die mit der Auffangschale verbundene Fixiereinlage liegt dann zwischen den Beinen der Patientin am Körper an und folgt dessen Konturen. Dadurch stellt die Fixiereinlage eine wesentlich größere Fläche zum Halten der gesamten Versorgungsvorrichtung zur Verfügung, als die Auffangschale alleine. Genau hier liegt der große Vorteil der Fixiereinlage: Beim anschließenden Anziehen der Unterhose wird die Versorgungsvorrichtung von der Unterhose entlang der gesamten Fläche der Fixiereinlage gehalten und fixiert. Diese gegenüber der Ausgangsschale alleine deutlich vergrößerte Haltefläche bewirkt eine deutlich verbesserte Positionsfixierung der Versorgungsvorrichtung. Somit ist eine gute Abdichtung der Versorgungsvorrichtung auch bei Bewegungen der Patientin sichergestellt.

Bei einer bevorzugten Ausgestaltung des Vorschlags ist vorgesehen, dass die Fixiereinlage zwischen den Oberschenkeln im Schritt der Trägerin einbringbar ist wobei ein breiterer Bereich der Fixiereinlage im unteren Bauchbereich der Trägerin anbringbar und ein schmalerer Bereich der Fixiereinlage im Anusbereich der Trägerin anbringbar ist. Der nach vorne orientierte Teil der Fixiereinlage ist breiter ausgeformt als der nach hinten orientierte Teil. Dadurch ist eine Verdrehsicherung der Fixiereinlage und somit der Versorgungsvorrichtung gegeben.

Des Weiteren ist vorgesehen, dass die Auffangschale und die Fixiereinlage durch eine Schnappverbindung miteinander verbindbar sind. In dieser Ausführungsform ermöglicht eine Schnappverbindung ein einfaches und schnelles Verbinden und Trennen der Elemente Auffangschale und Fixiereinlage. Dadurch wird das Anlegen der Versorgungsvorrichtung vereinfacht. So kann beispielsweise zunächst die Auffangschale alleine zwischen den Schamlippen eingebracht werden, anschließend wird dann die Fixiereinlage an der Auffangschale mithilfe der Schnappverbindung befestigt. Wäre die Fixiereinlage dauerhaft fest an der Ausgangsschale befestigt, würde sie unter Umständen die Sicht auf den Körper beim Einbringen der Auffangschale versperren. Selbstverständlich sind auch andere Verbindungen zwischen Auffangschale und Fixiereinlage mit zur Erfindung gehörend, wie beispielsweise Splintverbindungen, Bajonettverbindungen oder ähnliches. Auch eine einteilige, nicht trennbare Ausführung von Auffangschale und Fixiereinlage ist ebenfalls denkbar.

In einer vorteilhaften Ausgestaltung ist vorgesehen, dass die Fixiereinlage die Position und Ausrichtung der Auffangschale zum Harnröhrenausgang der Trägerin festlegt. Bei eingesetzter oder angebrachte Versorgungsvorrichtung liegt die Unterhose flächig an der nach unten gerichteten Oberfläche der Fixiereinlage an. Die Unterhose richtet somit die Fixiereinlage zum Körper der Patientin aus. Durch die lösbar aber dennoch feste Verbindung zwischen Fixiereinlage und Auffangschale bestimmt die Ausrichtung der Fixiereinlage wiederum die Position und Ausrichtung der Auffangschale zum Harnröhrenausgang der Trägerin.

Geschickter Weise ist vorgesehen, dass die Fixiereinlage an die Anatomie der Trägerin anpassbar ist. Besonders günstige Ergebnisse werden dadurch erzielt, dass die für die Ausrichtung der Versorgungsvorrichtung ausschlaggebende Fixiereinlage individuell an die Anatomie der Trägerin anpassbar ist. Wie bereits beschrieben ist die Fixiereinlage aus einem steifen Material gefertigt, welches allerdings in gewissem Rahmen elastisch verformbar ist und somit auch den Bewegungen des Körpers der Patientin folgen kann. Eine individuelle Anpassbarkeit kann vorgenommen werden, indem die Fixiereinlage in ihrer Grundwölbung veränderbar ist. Beispielsweise kann die Fixiereinlage so ausgeführt sein, dass sie vor der ersten Benutzung an den Körper der Patientin angelegt wird und somit die Wölbung des Körpers auf die Fixiereinlage Übertragen wird. Diese individuelle Wölbung wird dann fixiert, beispielsweise durch Erhitzen der Fixiereinlage. Nach dieser Fixierung behält dann die Fixiereinlage die individuell angepasste Grundform, bleibt jedoch aus dieser Grundform heraus elastisch. Eine solche Ausführung kann beispielsweise über Werkstoffe geschaffen werden, die über eine Aktivierung, beispielsweise ein Erhitzen, in ihrer Grundform festgelegt werden können. Eine einfachere Ausführungsform mit einem ähnlichen Konzept ist durch eine Fixiereinlage aus einem Metallblech möglich. Dieses Metallblech lässt sich plastisch an die Form des Körpers anpassen, weist aber dann auch eine elastische Verformbarkeit aus der Grundform heraus auf. Neben einer individuellen Anpassbarkeit der Wölbung der Fixiereinlage kann auch deren äußere Form und Größe anpassbar gestaltet sein. In der Draufsicht weist die Fixiereinlage eine im wesentlichen dreieckige Form auf. Für unterschiedliche Körper sind unterschiedliche Abmessungen dieser dreieckigen Grundform optimal. Eine Anpassbarkeit kann beispielsweise dadurch erreicht werden, dass die Fixiereinlage zunächst sehr groß hergestellt wird. Ist der Körper der Patientin dann kleiner als die Fixiereinlage, können deren Ränder einfach mit einer Schere abgeschnitten werden so lange, bis die Form der Fixiereinlage optimal zum Körper der Patientin passt. Neben einer Zuschneidbarkeit können auch Sollbruchstellen an den Rändern der Fixiereinlage vorgesehen werden, entlang der die Form durch Abbrechen nicht benötigter Ränder eingestellt werden kann. Alternativ kann auch zunächst ein Abdruck oder eine optische Vermessung des Körpers der Patientin vorgenommen werden und anschließend die Fixiereinlage basierend auf den ermittelten Abmessungen individuell hergestellt werden.

In einer weiteren bevorzugten Ausführungsform ist vorgesehen, dass die Fixiereinlage aus einem steiferen Material als die Auffangschale besteht. In dieser Ausführungsform ist das Material der Fixiereinlage steifer als das Material der Auffangschale ausgeführt. Für die Ausgangsschale ist eine elastische Flexibilität besonders wichtig, um eine dauerhaft sichere Abdichtung des Harnröhrenausganges gegenüber der Umgebung sicherzustellen. Die Fixiereinlage dagegen funktioniert optimal, wenn sie eine sichere, fest mit der Unterhose verbundene Basis für die flexible Auffangschale bereitstellt. Eine solche Basis ist durch ein steifes Material der Fixiereinlage besonders gut gegeben.

Des Weiteren ist günstiger Weise vorgesehen, dass das Urinsammelelement einen Vaginalanker aufweist, der in die Vagina der Trägerin einführbar ist um das Urinsammelelement zusätzlich zu fixieren, wobei der Vaginalanker im Wesentlichen walzenförmig ausgebildet ist. In dieser Ausführungsform ist zusätzlich ein Vaginalanker vorgesehen, der zum Urinsammelelement gehört. Dieser Vaginalanker wird beim Anbringen der Versorgungsvorrichtung in die Vagina der Trägerin eingeführt. Dadurch wird die gesamte Versorgungsvorrichtung und insbesondere das Urinsammelelement zusätzlich zum Körper der Trägerin fixiert. Der Vaginalanker verhindert vor allem ein verrutschen des Urinsammelelementes aus seiner Position zwischen den Schamlippen. Besonders günstig hat sich ein Vaginalanker erwiesen, welcher im wesentlichen walzenförmig oder zylindrisch ausgeführt ist. Auch eine kegelförmige Form des Vaginalankers, bei der der in die Vagina hineinweisende Teil des Vaginalankers breiter als der zum Urinsammelelement weisende Teil ausgeführt ist hat sich als günstig herausgestellt. Durch den breiteren, im Inneren der Vagina befindlichen Teile des Vaginalankers wird ein Herausrutschen des Vaginalankers und damit ein Entfernen der Versorgungsvorrichtung vom Körper der Patientin besonders gut verhindert. Ein Vaginalanker kann entweder in Kombination mit der weiter oben beschriebenen Fixiereinlage oder auch als Alternative zu dieser Fixiereinlage verwendet werden.

Vorteilhafter Weise ist vorgesehen, dass der Vaginalanker in einem Winkel α zur von der Auffangöffnung aufgespannten Ebene angeordnet ist. Der Winkel zwischen der Mittelachse des bevorzugt walzenförmig ausgeführten Vaginalankers Zu der Ebene, welche von der Aufangöffnung der Ausgangsschale aufgespannt wird, wird als Winkel α bezeichnet. Da die obere Begrenzung der Scheide und die Mitte der Vagina in einem Winkel von größenordnungsmäßig 70° zueinander verlaufen, ist auch ein Winkel von 70° ein guter Grundwert für den Winkel α. Allerdings sind auch andere davon abweichende Winkel mit zur Erfindung gehörend. Durch eine sinnvolle Auswahl eines Winkels α wird sichergestellt, dass die Aufnahmeöffnung optimal zu dem Körperbereich ausgerichtet ist, welcher den Ausgang der Harnröhre umgibt. Wesentlich für die Funktion der Versorgungsvorrichtung ist ein dichtes Anliegen der Auffangschale in diesem Bereich. Die zur Fixierung der Versorgungsvorrichtung vorgesehenen Elemente, wie der Vaginalanker, sind so auszuführen, dass stets eine gute Abdichtung gegeben ist. Es ist denkbar, zunächst die Abmessungen des Körpers einer Patientin zu ermitteln, insbesondere Abstand und Winkel von Vagina zum oberen Rand der Scheide, und anschließend individuell basierend auf den ermittelten Abmessungen eine optimale Versorgungsvorrichtung herzustellen.

Des Weiteren ist in dem Vorschlag vorteilhafter Weise vorgesehen, dass das Urinsammelelement aus Gummi, Latex, PVC,... besteht. Für die Funktion des Urinsammelelementes ist es wichtig, dass es aus einem Material besteht, welches gegenüber Haut und Schleimhaut gut abdichtet. Weiterhin ist es wichtig, dass das Material elastisch ist und sich dem Körper sowie Körperbewegungen gut anpassen kann. Geeignete Materialien sollten darüber hinaus gut zu reinigen bzw. zu sterilisieren sein die notwendige Hygiene bei solchen Versorgungsvorrichtungen sicherstellen zu können. Sämtliche Materialien, die diese Anforderungen erfüllen sind als mit zur Erfindung gehörend zu betrachten.

Bei einer bevorzugten Ausgestaltung des Vorschlags ist vorgesehen, dass das kajakförmig ausgeführte Urinsammelelement zumindest zwei Halteflügel aufweist, welche seitlich am Urinsammelelement angeordnet sind und welche nicht innerhalb der Schamlippen der Trägerin einbringbar sind und welche im Bereich zwischen den Schamlippen und den Innenseiten der Oberschenkel der Trägerin einbringbar sind. In dieser alternativen Ausführungsform einer Versorgungsvorrichtung ist das Urinsammelelement kajakförmig ausgeführt. Eine Abdichtung zwischen dem Urinsammelelement und dem Körper der Trägerin erfolgt nicht, wie in den zuvor beschriebenen Ausführungsformen, im Inneren der Scheide, also zwischen den Schamlippen, sondern außerhalb der Schamlippen in einem Bereich zwischen den Schamlippen und dem Bein. Die kajakförmige Ausführungsform hat den Vorteil, dass keine Teile der Vorrichtung innerhalb der Schamlippen eingeführt werden müssen. Manchen Patientinnen ist eben dieses Einbringen eines Fremdkörpers ins Innere der Scheide unangenehm. Für eine Positionierung des kajakförmigen Urinsammelelementes weist dieses an seinen Seiten zumindest zwei Halteflügel auf. Diese Halteflügel stützen sich an den Innenseiten der Beine bzw. Oberschenkel der Trägerin ab. Ein kajakförmiges Urinsammelelement wird analog zu der oben beschriebenen Ausführungsform zusammen mit einer Unterhose und einem Fixierelement verwendet. Somit ist auch das sich außerhalb der Scheide befindende kajakförmige Urinsammelelement durch die anderen Elemente der Versorgungsvorrichtung bestens positioniert und gegen Verdrehung geschützt.

Des Weiteren ist vorgesehen, dass das kajakförmig ausgeführte Urinsammelelement eine Dichtfläche aufweist, welche am oberen Rand des Urinsammelelementes angeordnet ist und bei verwendeter Versorgungsvorrichtung außerhalb der Schamlippen der Trägerin anliegt. Diese Ausführungsform hat den Vorteil, dass bei der Verwendung der Versorgungseinrichtungen kein Fremdkörper zwischen die Schamlippen eingeführt werden muss. Dies kann Vorteile beim Tragekomfort haben. Eine Abdichtung erfolgt in diesem Fall zwischen einer Dichtfläche am oberen Rand des Urinsammelelementes und der Haut außerhalb der Schamlippen der Trägerin.

In einer vorteilhaften Ausgestaltung ist vorgesehen, dass das Urinsammelelement die Form eines Kajaks aufweist, welches bereichsweise zwischen den Schamlippen der Trägerin anbringbar ist. In dieser Ausführungsform werden die Vorteile eines kajakförmigen Urinsammelelementes, welches sich größtenteils außerhalb der Schamlippen befindet, mit den Vorteilen eines sich zwischen den Schamlippen befindenden Urinsammelelementes verknüpft. Eine Anbringung der Versorgungsvorrichtung außerhalb der Scheide fühlt sich für einige Patientinnen angenehmer an, als eine Anbringung im Inneren der Scheide. In der hier beschriebenen Ausführungsform befindet sich der Großteil der Versorgungsvorrichtung außerhalb des Körpers. Lediglich ein kleiner Bereich des Urinsammelelementes wird zwischen den Schamlippen platziert. Die Abdichtung gegen Herauslaufen von Urin ist erfahrungsgemäß im Inneren der Scheide einfacher sicherzustellen. Die im Inneren der Scheide angeordneten Bereiche des Urinsammelelementes dienen lediglich der Abdichtung. Die Funktionen des Haltens und Fixierens der Versorgungsvorrichtung werden dagegen von Bereichen oder Elementen übernommen, welche außerhalb der Scheide bzw. der Schamlippen angeordnet sind.

Die Aufgabe der Erfindung wird ebenfalls gelöst durch Set umfassend
- eine Versorgungsvorrichtung nach einem der vorhergehend beschriebenen Ausführungsformen
- und Mittel zur positionsgenauen Anbringung der Durchführung in der Unterhose.

Ein erfindungsgemäßes Set ermöglicht es, eine optimal an den Körper einer Patientin angepasste Versorgungsvorrichtung zur Verfügung zu stellen. In seiner einfachsten Ausführungsform Umfasst das Set eine Versorgungsvorrichtung nach einer der zuvor beschriebenen Ausführungsformen. Diese Versorgungsvorrichtung ist in identischer Ausführungsteil eines jeden Sets. Zusätzlich zur Versorgungsvorrichtung umfasst das Set Mittel zur positionsgenauen Anbringung der Durchführung in der Unterhose. Die zur Versorgungsvorrichtung gehörende, mitgelieferte Unterhose wird zunächst ohne Durchführung bereitgestellt. Die Mittel dienen dann dazu, vor Ort und individuell die Position der Durchführung in der Unterhose anzubringen und so sicherzustellen, dass sich diese Durchführung in der optimalen Position befindet.

Bei einer bevorzugten Ausgestaltung des Vorschlags ist vorgesehen, dass die Mittel zur positionsgenauen Anbringung der Durchführung ein Markierungselement, insbesondere einen textilfesten Filzstift und ein Schneidelement, insbesondere einen Schneidstempel umfassen. Besonders einfach kann die Durchführung in der Unterhose in folgendem Vorgehen eingebracht werden: zunächst wird das Urinsammelelement und falls vorhanden die Fixiereinlage am Körper der Patientin positioniert, so dass die Urinableitung des Urinsammelelementes frei nach unten steht. Anschließend wird die Unterhose, welche noch keine Durchführung aufweist, angezogen. Die Urinableitung ist in der geschlossenen Unterhose deutlich zu erkennen. Nun wird mithilfe eines textilfesten Filzstift des die Position der Urinableitung auf der Unterhose markiert. Anschließend wird die Unterhose wieder ausgezogen und an der Stelle der Markierung die Durchführung eingebracht. Die Durchführung kann besonders einfach durch einen Schneidstempel, ähnlich einem Papierlocher, eingebracht werden. Nach dem Einbringen der Durchführung kann die Versorgungsvorrichtung wie weiter oben beschrieben verwendet werden. Soll die Unterhose ausgewechselt werden, kann eine neue Ersatzunterhose wieder mit dem oben beschriebenen Verfahren mit einer optimal positionierten Durchführung versehen werden.

In den Zeichnungen ist die Erfindung insbesondere in Ausführungsbeispielen schematisch dargestellt. Es zeigen:
- Fig. 1: eine geschnittene Seitenansicht durch eine erste Ausführungsform einer erfindungsgemäßen Versorgungsvorrichtung mit schematischer Darstellung des Körpers der Patientin,
- Fig. 2: eine Draufsicht auf einen Teil einer ersten Ausführungsform einer erfindungsgemäßen Versorgungsvorrichtung ohne Darstellung des Körpers der Patientin,
- Fig. 3: eine Draufsicht auf einen anderen Teil einer ersten Ausführungsform einer erfindungsgemäßen Versorgungsvorrichtung ohne Darstellung des Körpers der Patientin,
- Fig. 4: eine Draufsicht auf einen weiteren Teil einer ersten Ausführungsform einer erfindungsgemäßen Versorgungsvorrichtung ohne Darstellung des Körpers der Patientin,
- Fig. 5: eine perspektivische Ansicht einer Ausführungsform eines erfindungsgemäßen Urinsammelelementes,
- Fig. 6: eine geschnittene Seitenansicht durch eine zweite Ausführungsform einer erfindungsgemäßen Versorgungsvorrichtung mit schematischer Darstellung des Körpers der Patientin,
- Fig. 7: eine Draufsicht auf einen Teil einer zweiten Ausführungsform einer erfindungsgemäßen Versorgungsvorrichtung ohne Darstellung des Körpers der Patientin,
- Fig. 8: eine perspektivische Ansicht einer weiteren Ausführungsform eines erfindungsgemäßen Urinsammelelementes.

In den Figuren sind gleiche oder einander entsprechende Elemente jeweils mit den gleichen Bezugszeichen bezeichnet und werden daher, sofern nicht zweckmäßig, nicht erneut beschrieben. Die in der gesamten Beschreibung enthaltenen Offenbarungen sind sinngemäß auf gleiche Teile mit gleichen Bezugszeichen bzw. gleichen Bauteilbezeichnungen übertragbar. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiterhin können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

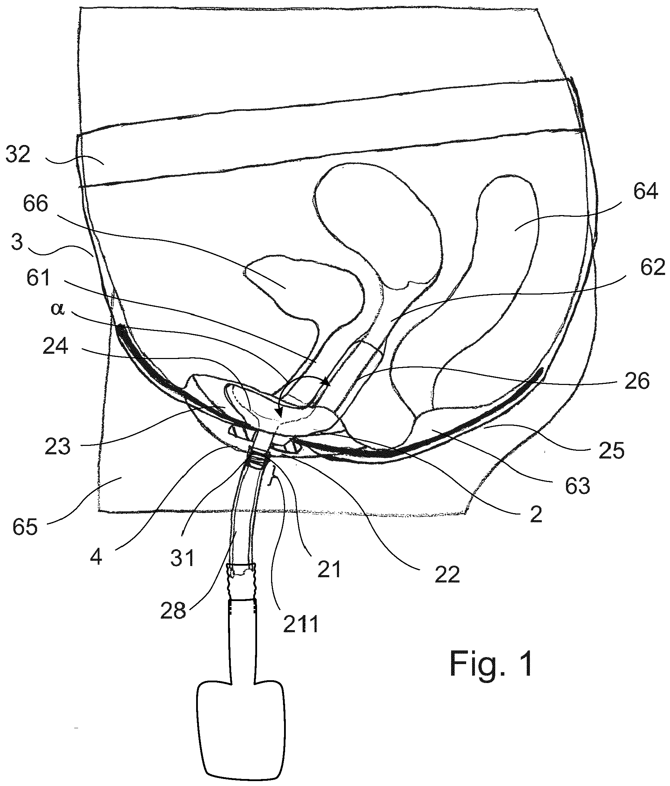

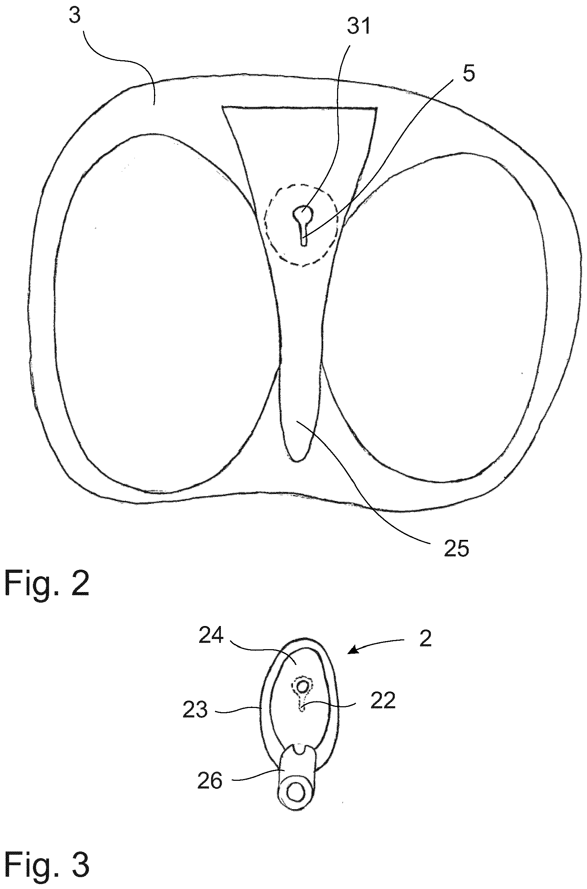

Fig. 1 zeigt eine geschnittene Seitenansicht durch eine erste Ausführungsform einer erfindungsgemäßen Versorgungsvorrichtung mit schematischer Darstellung des Körpers der Patientin. Der Körper einer Patientin ist von der Seite geschnitten dargestellt. Der Bauchnabel der Patientin weist dabei nach links. Im unteren Bereich ist der obere Teil des rechten Oberschenkels 65 der Patientin zu sehen. Das linke Bein befindet sich vor der Zeichenebene und ist somit nicht zu sehen. Die inneren Organe im Unterleib der Patientin sind soweit für das Verständnis der Erfindung erforderlich dargestellt. Der Urin wird im Körper in der Blase 66 gespeichert und durch die Harnröhre 61 aus dem Körper befördert. Bei Inkontinenz dichtet die Harnröhre 61 nicht sicher gegen Ausfluss von Urin ab. Es ist somit Aufgabe der erfindungsgemäßen Versorgungsvorrichtung aus dem unteren Ende der Harnröhre 61 austretenden Urin aufzufangen. Rechts, annähernd parallel zur Harnröhre 61, verläuft die Vagina 62. Noch weiter rechts ist der Dickdarm 64 mit seinem Ausgang, dem Anus 63 zu sehen. Zu der Versorgungsvorrichtung gehört als zentrales Element das Urinsammelelement 2. Das Urinsammelelement 2 weist eine Auffangschale 23 auf. Das Urinsammelelement 2 ist ungeschnitten dargestellt, das Innere der Auffangschale 23 ist gestrichelt dargestellt. Es ist klar zu erkennen, dass aus dem unteren Ende der Harnröhre 61 austretender Urin in das gestrichelt dargestellte Innere der Auffangschale 23 läuft und dort nach unten geleitet wird. An die Auffangeschale 23 schließt nach unten die Urinableitung 21 an. Die Urinableitung 21 dient der Abführung und der Wegführung des Urins vom Körper. Im unteren Bereich der Urinableitung 21 befindet sich ein Anschlussbereich 211, in dem ein Abflussschlauch 28 befestigt ist. Im vorliegenden Fall ist der Anschlussbereich 211 gerippt ausgebildet. Der Anschlussbereich 211 weitet durch diese Rippen den Abflussschlauch 28 auf. Die dadurch im Abflussschlauch 28 entstehenden elastischen Rückstellkräfte sorgen für einen sicheren und dichten Halt des Schlauchendes des Abflussschlauches 28 auf dem Anschlussbereich 211. Der Abflussschlauch 28 führt zu einem Urinsammelbehälter, beispielsweise einem Sammelbeutel, der am Bein befestigt ist. Am unteren Rand der Auffangschale 23 ist lösbar fest die Fixiereinlage 25 befestigt. Die Fixiereinlage 25 ist im Schnitt geschwärzt dargestellt und erstreckt sich vom Unterbauch der Patientin links bis über den Anus 63 hinaus nach rechts. Die Fixiereinlage 25 dient einer sicheren Positionierung und Ausrichtung des Urinsammelelementes 2 zur Scheide und dem Harnröhrenausgang 61. In der dargestellten geschnittenen Seitenansicht ist die Fixiereinlage 25 gewölbt und verläuft entlang der Körperkontur der Patientin im Schritt. Die Fixiereinlage 25 kann dabei so ausgeführt sein, dass deren Wölbung individuell an den Körper anpassbar ist. Die Verbindung von Auffangschale 23 und Fixiereinlage 25 erfolgt hier über einen nicht näher dargestellte Schnappverbindung. Damit sind die beiden Elemente ohne Werkzeug voneinander lösbar und wieder miteinander verbindbar. Mit zur Versorgungsvorrichtung gehört auch die Unterhose 3. Die Unterhose 3 ist, soweit nicht anders beschrieben, wie eine konventionelle Unterhose ausgeführt. Erfindungsgemäß weist die Unterhose jedoch eine Durchführung 31 auf, durch welche die Urinableitung 21 des Urinsammelelementes 2 geführt wird. Die Durchführung 31 kann dabei beispielsweise als kreisrundes Loch in der Unterhose 3 ausgeführt sein. Das Urinsammelelement 2 und die Unterhose 3 sind somit miteinander verbunden, wodurch die Unterhose dazu genutzt werden kann, auch das Urinsammelelement 2 am Körper der Patientin zu fixieren. Um diese Fixierung weiter zu verbessern kann die Unterhose 3 einen oder auch mehrere Haltebünde 32 aufweisen. Im dargestellten Fall weist die Unterhose 3 an deren oberen Rand einen solchen Haltebund 32 auf. Dieser ist fester und mit einer höheren elastischen Rückstellkraft ausgeführt, als der restliche Stoff der Unterhose. Dadurch liegt der Haltebund 32 besonders fest am Körper an. Zusätzlich ist der Haltebund auf seiner inneren, zum Körper hin weisenden Seite mit einem besonders rutschfesten Material beschichtet, beispielsweise einer speziellen Gummimischung. Dadurch wird zusätzlich ein sicherer Halt der Unterhose am Körper gewährleistet. Beim Anbringen der Versorgungsvorrichtung am Körper wird zunächst das Urinsammelelement 2 so positioniert, dass die Auffangschale 23 unterhalb des Ausgangs der Harnröhre 61 platziert ist. Anschließend wird die Unterhose angezogen, wobei die Urinableitung 21 durch die Durchführung 31 geführt wird. Zur Befestigung des Urinsammelelementes 2 an der Unterhose 3 ist zusätzlich das Fixierelement 4 vorgesehen, welches auf der Urinableitung 21 angebracht wird. Im vorliegenden Fall ist das Fixierelement 4 als Ring ausgebildet, der in der Schnittansicht gestrichelt dargestellt ist. Das Fixierelement 4 sitzt fest auf der Urinableitung und klemmt die Unterhose zwischen der Auffangschale 23 und dem Fixierelement 4 ein. Dadurch ist sichergestellt, dass das Urinsammelelement 2 und die Unterhose 3 fest miteinander verbunden sind und weder ein Verschieben, noch ein Verdrehen des Urinsammelelementes 2 zur Unterhose 3 möglich ist. Dadurch ist ein sicherer Sitz der Versorgungsvorrichtung am Körper der Patientin gewährleistet. Um die Verdrehsicherheit des Urinsammelelementes 2 gegenüber der Unterhose 3 bzw. der Durchführung 31 weiter zu verbessern weist das Urinsammelelement 2 in der dargestellten Ausführungsform eine Positionsführung 22 auf. Diese Positionsführung 22 ist als schmaler Flügel ausgeführt, welcher sich vertikal nach unten erstreckt. Die Durchführung 31 in der Unterhose 3 weist eine, der Form der Positionsführung 22 entsprechende Führungsaufnahme 5 auf. Das Zusammenspiel dieser beiden Formen bewirkt einen Formschluss, der ein Verdrehen des Urinsammelelementes 2 gegenüber der Unterhose 3 verhindert. Die längliche, flügelartige Form der Positionsführung 22 wird in einer ebenso länglichen Führungsaufnahme 5 aufgenommen. Um das Fixierelement 4 über der Positionsführung 22 befestigen zu können, weist auch das Fixierelement 4 hier eine Führungsaufnahme 5 auf. Somit ist das Fixierelement 4 kollisionsfrei mit der Positionsführung 22 auf der Urinableitung 21 anbringbar. Das Zusammenspiel von Positionsführung 22 und Führungsaufnahme 5 ist auch in Fig. 2 und 3 dargestellt. Das Urinsammelelement 2 weist weiterhin einen Vaginalanker 26 auf, der im hinteren Bereich der Auffangschale 23 angeordnet ist. Der Vaginalanker 26 dient der Verbesserung der Fixierung des Urinsammelelementes 2 zum Körper der Patientin. Der Vaginalanker 26 ist hier walzenförmig ausgeführt und weist eine Länge von ca. 10 cm auf. Beim Anlegen der Versorgungsvorrichtung wird der Vaginalanker 26 in die Vagina 62 eingeführt. Dadurch wird die Auffangöffnung 24 der Auffangschale 23 so positioniert, dass sie den Ausgang der Harnröhre 61 umschließt und gleichzeitig dicht an der Schleimhaut um den Harnröhrenausgang herum anliegt. Der Vaginalanker 26 ist mit seiner Mittelachse in einem Winkel α zur von der Auffangöffnung 24 auf gespannten Ebene angeordnet. Das Urinsammelelement 2 kann dabei so ausgeführt sein, dass dieser Winkel α unveränderlich ist. Es ist aber auch möglich den Vaginalanker 26 biegsam auszuführen und so eine Anpassbarkeit des Winkels α zu ermöglichen. Durch einen veränderlichen Winkel α ist eine optimale Anpassung der Versorgungsvorrichtung an den individuellen Körper einer Patientin möglich.
Fig. 2 zeigt eine Draufsicht auf einen Teil einer ersten Ausführungsform einer erfindungsgemäßen Versorgungsvorrichtung ohne Darstellung des Körpers der Patientin. In Fig. 2 ist die Unterhose 3 von oben zu sehen. Deutlich zu erkennen sind die beiden ovalen Öffnungen, durch welche die Beine geführt werden. Zwischen den Beinen angeordnet ist die Fixiereinlage 25. In dieser Draufsicht ist deutlich zu erkennen, dass die Fixiereinlage 25 eine im Wesentlichen dreieckige Grundform aufweist. Der breitere, in der Zeichnung oben dargestellte Teil wird dabei am Unterbauch bzw. am Schamdreieck der Patientin angelegt, der spitze in der Zeichnung nach unten weisende Teil wird im Anusbereich angelegt. In der Mitte des gestrichelten Kreises in der Fixiereinlage 25 ist die Durchführung 31 als kreisrundes Loch dargestellt. Die Durchführung 31 ist in der Unterhose 3 angebracht. Die Fixiereinlage 25 ist lösbar fest mit dem Urinsammelelement 2 verbindbar, welches in Fig. 3 in getrenntem Zustand von der Fixiereinlage 25 dargestellt ist. Um ein Durchdringen der Urinableitung 21 auch durch die Fixiereinlage 25 Zu ermöglichen, weist auch diese eine in der Form der Durchführung 31 der Unterhose 3 entsprechende Durchführung auf. Nach unten weg weisend von dieser Durchführung in der Fixiereinlage 25 und der Durchführung 31 in der Unterhose 3 ist die Führungsaufnahme 5 dargestellt, welche länglich ausgeführt ist. Zusammen mit der Durchführung sieht die Führungsaufnahme 5 aus wie ein Schlüsselloch. Bei der Montage der Fixiereinlage 25 mit dem Urinsammelelement 2 wird in die Führungsaufnahme 5 die Positionsführung 22 des Urinsammelelementes 2 eingeführt. Diese beiden Elemente wirken nun nach dem Schlüssel-Schlossprinzip zusammen und verhindern in Kombination formschlüssig ein Verdrehen und Verrutschen des Urinsammelelementes 2 und der Fixiereinlage 25 gegenüber der Unterhose 3.
Fig. 3 zeigt eine Draufsicht auf einen anderen Teil einer ersten Ausführungsform einer erfindungsgemäßen Versorgungsvorrichtung ohne Darstellung des Körpers der Patientin. Der Maßstab und die Perspektive in Fig. 3 ist gleich zu Fig. 2. In Fig. 3 ist in einer Draufsicht das Urinsammelelement 2 dargestellt, welches auf der in Fig. 2 dargestellten Fixiereinlage 25 montiert wird. Von oben ist beim Urinsammelelement 2 in Fig. 3 die Auffangschale 23 mit der nach oben orientierten Auffangöffnung 24 zu sehen. Der Rand der Auffangöffnung 24 liegt im eingebrachten Zustand der Versorgungsvorrichtung oben an der Scheidenwand dicht an. Unten in Fig. 3 ist der vaginale Anker 26 zu sehen, welcher sich schräg nach oben aus der Zeichnungsebene heraus erstreckt. Der Vaginalanker 26 ist im dargestellten Fall rohrförmig, d.h. innen hohl ausgeführt. Dies ermöglicht das Abfließen von Sekret oder anderen Flüssigkeiten aus der Vagina hinaus, wobei diese Flüssigkeit dann von der Auffangschale 23 aufgenommen und abgeleitet wird. Im obersten Drittel der Auffangschale 23 ist die Öffnung für die Urinableitung 21 zu sehen. Gestrichelt um diese Öffnung herum findet sich wieder eine schlüssellochartige Form, wie sie schon aus Fig. 2 bekannt ist. Unten an der Auffangschale 23 ist die Positionsführung 22 angebracht, welche bei der Montage mit der Fixiereinlage 25 in deren Führungsaufnahme 5 gesteckt wird.

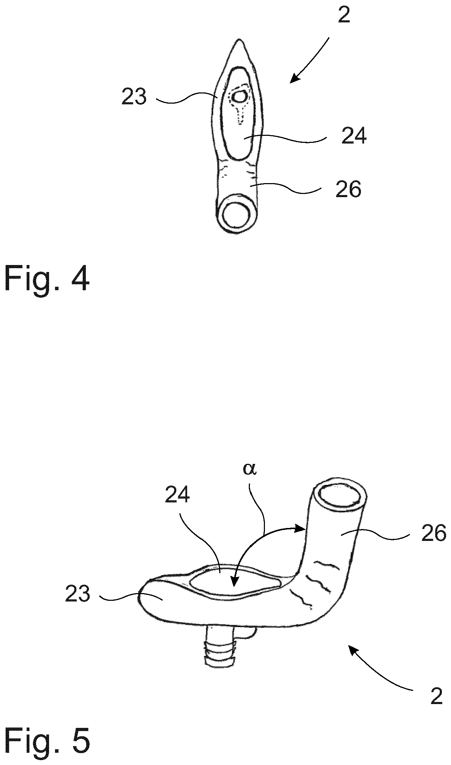

Fig. 4 zeigt eine Draufsicht auf einen weiteren Teil einer ersten Ausführungsform einer erfindungsgemäßen Versorgungsvorrichtung ohne Darstellung des Körpers der Patientin. In Fig. 4 wird ein Urinsammelelement 2 in einer identischen Draufsicht gezeigt wie das Urinsammelelement 2 in Fig. 3. Das Urinsammelelement 2 in Fig. 4 weist eine schmälere Auffangschale 23 auf. Das in Fig. 4 dargestellte Urinsammelelement 2 ist aus einem Schlauch hergestellt, welcher zunächst einen konstanten zylindrischen Durchmesser aufweist. Ein Teil dieses Schlauches bildet nach Fertigstellung des Urinsammelelementes 2 den Vaginalanker 26. Der vordere Teil des Schlauches bildet die Auffangschale 23 mit der Auffangöffnung 24. Die Auffangöffnung 24 entsteht dadurch, dass von dem ursprünglich geschlossenen Schlauch ein Teil des Umfangs entfernt wird. An der Spitze ist der Schlauch zusammengeschweißt um eine dichte Auffangschale sicherzustellen. Die dargestellte Ausführungsform eines Urinsammelelementes 2 ist besonders einfach und kostengünstig herzustellen.
Fig. 5 zeigt eine perspektivische Ansicht einer Ausführungsform eines erfindungsgemäßen Urinsammelelementes. Dargestellt ist hier die Ausführungsform, welche in Fig. 4 in einer Draufsicht zu sehen ist. In der perspektivischen Ansicht wird besonders gut deutlich, dass das Urinsammelelement 2 aus einem Schlauch mit konstantem Durchmesser gefertigt wurde. Zur Erzielung des Winkels α zwischen dem Vaginalanker 26 und der Auffangöffnung 24 im Inneren des Schlauches ein Metalldraht vorgesehen sein, der zur Anpassung des Winkels α von Hand plastisch verformbar ist, nach der Anpassung aber stabil seine Form behält.

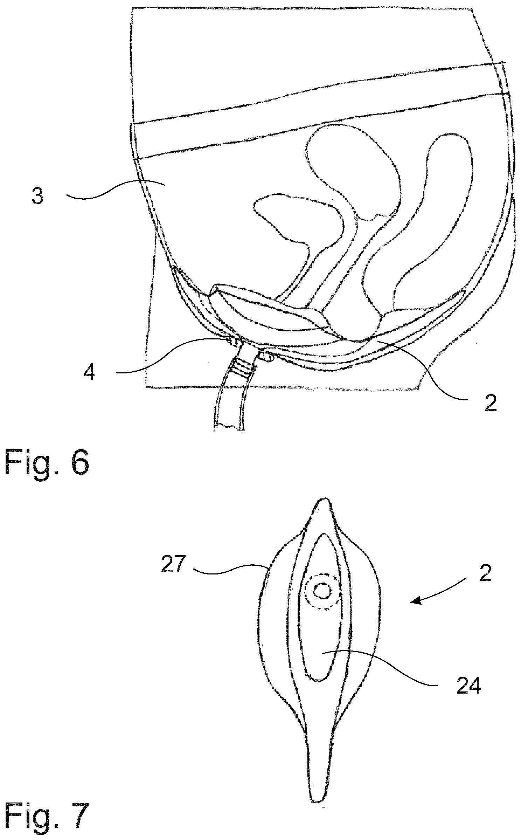

Fig. 6 eine geschnittene Seitenansicht durch eine zweite Ausführungsform einer erfindungsgemäßen Versorgungsvorrichtung mit schematischer Darstellung des Körpers der Patientin. In Fig. 6 ist eine weitere Ausführungsform einer erfindungsgemäßen Versorgungsvorrichtung dargestellt. Die Darstellung des Körpers der Patientin sowie der Unterhose 3 entspricht der Darstellung in Fig. 1. Die in Fig. 6 dargestellte Ausführungsform unterscheidet sich von der in den vorhergehenden Figuren dargestellten Ausführungsform dadurch, dass keine Elemente des Urinsammelelementes 2 in die Scheide oder zwischen die Schamlippen eingeführt werden. Die Abdichtung des Urinsammelelementes 2 zum Körper der Patientin erfolgt in dem Bereich zwischen äußeren Schamlippen und Bein. Das Urinsammelelement 2 ist dazu kajakförmig ausgeführt. Auch das dargestellte Urinsammelelement 2 weist eine Urinableitung 21 auf, welche durch eine Durchführung 31 in der Unterhose 3 geführt wird und auf der gegenüberliegenden Seite der Unterhose 3 mit einem Fixierelement 4 fest positioniert wird.
Fig. 7 zeigt eine Draufsicht auf einen Teil einer zweiten Ausführungsform einer erfindungsgemäßen Versorgungsvorrichtung ohne Darstellung des Körpers der Patientin. Dargestellt ist hier in einer Draufsicht das Urinsammelelement 2 aus Fig. 6. Sein oben ist deutlich die Kajakform zu erkennen, welche eine Auffangöffnung 24 aufweist. Die Auffangöffnung 24 ist hier größer ausgeführt als in der zuvor dargestellten Ausführungsform, da sie außerhalb der Schamlippen anliegt. Seitlich am Urinsammelelement 2 sind zwei Halteflügel 27 angebracht, welche der besseren Fixierung des Urinsammelelementes 2 am Körper dienen. Die beiden Halteflügel 27 liegen mit ihrem äußeren Rand an der Innenseite des Oberschenkels der Patientin an. Dadurch wird das kajakförmige Urinsammelelement 2 zwischen den Beinen der Patienten festgeklemmt. Wie in Fig. 6 dargestellt, erfolgt zusätzlich eine Verbindung des Urinsammelelementes 2 mit der Unterhose 3 und dem Fixierelement 4. Dadurch ist eine dichte und sichere Fixierung der Versorgungsvorrichtung auch in dieser zweiten Ausführungsform sichergestellt.

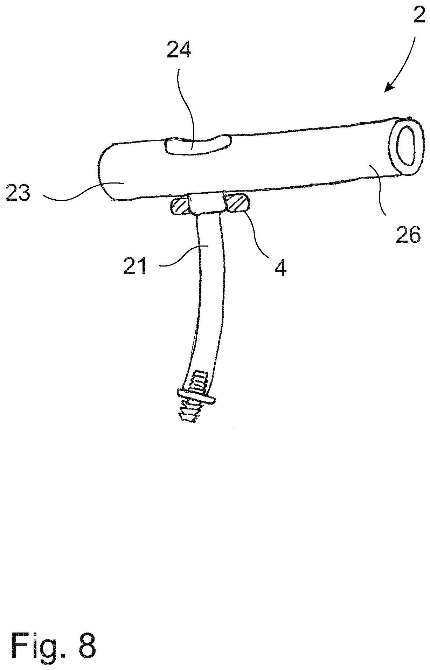

Fig. 8 zeigt eine perspektivische Ansicht einer weiteren Ausführungsform eines erfindungsgemäßen Urinsammelelementes. Fig. 8 zeigt eine weitere Ausführungsform, welche einige alternative zu den in Fig. 3, 4 und 5 dargestellten Ausführungsformen aufweist. Zu sehen ist ein Urinsammelelement 2 auf welches im unteren Bereich ein ringförmiges Fixierelement 4 aufgesteckt ist. Die gezeigte Ausführungsform ist selbstverständlich auch in Kombination mit einer Fixiereinlage 25 zu verwenden. Auch die in anderen Figuren dargestellte Unterhose 3 ist aus Gründen der Übersichtlichkeit hier nicht dargestellt. Eine Fixiereinlage 25 und die Unterhose 3 würden in der dargestellten Ausführungsform zwischen dem Urinsammelelement 2 und dem Fixierelement 4 eingeklemmt. Somit gelten die zuvor beschriebenen Vorteile der anderen Ausführungsformen auch für die in Fig. 8 dargestellte Ausführungsform. Das Urinsammelelement 2 weist hier einen Vaginalanker 26 auf. Urinsammelelement 2 und der Vaginalanker 26 bilden hier zusammen ein zylindrisches oder walzenförmiges Element. Das nach rechts gewandte Ende weist hier eine Öffnung auf welche im Inneren des Urinsammelelementes 2 in die Auffangschale 23 mündet. Die nach rechts gewandte Seite des Vaginalankers 26 kann auch geschlossen ausgeführt sein. Die gezeigte Ausführungsform kann elastisch verformbar sein, so dass der Vaginalanker 26 zur Auffangöffnung 24 biegsam ist. Es ist aber auch eine starre Ausführung der Kombination aus Vaginalanker 26 und Urinsammelelement 2 denkbar. Die Urinableitung 21 ist hier als, im Vergleich zu den anderen Ausführungsformen längerer Schlauch ausgebildet. Am Ansatz der Urinableitung 21 am Urinsammelelement 2 ist ein Knickschutz vorgesehen, welcher gleichzeitig als Sitz für das Fixierelement 4 dient. Der Durchmesser der Urinableitung 21 ist im Bereich dieses Knickschutzes größer ausgeführt. Der Knickschutz kann auch durch ein separates Teil, beispielsweise durch eine aus Draht gebildete Röhre ausgeführt sein. Der Knickschutz sorgt dafür, dass die Urinableitung 21 bei Bewegungen der Trägerin nicht geknickt und abgeklemmt wird und so den Ablauf des Urins verhindert wird. Die verlängerte Urinableitung 21 ist als flexibler Schlauch ausgeführt. Durch diese flexible Ausführung wird der Tragekomfort verbessert, da die Patienten keine harten Teile in der Scheide oder in unmittelbarer Nachbarschaft dazu spürt. Eine Verbindung mit einem nicht dargestellten Abflussschlauch 28 erfolgt über einen ganz unten dargestellten Steckverbinder. Diese Verbindung liegt dann irgendwo zwischen den Oberschenkeln der Patientin und wird an dieser Stelle deutlich weniger störend empfunden als im unmittelbaren Scheidenbereich. Die in Fig. 8 dargestellten Merkmale sind selbstverständlich auch mit Ausführungsformen kombinierbar, welche in den anderen Figuren dargestellt sind.

Die jetzt mit der Anmeldung und später eingereichten Ansprüche sind ohne Präjudiz für die Erzielung weitergehenden Schutzes.

Sollte sich hier bei näherer Prüfung, insbesondere auch des einschlägigen Standes der Technik, ergeben, dass das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist. Auch eine solche Unterkombination ist von der Offenbarung dieser Anmeldung abgedeckt.

Es ist weiter zu beachten, dass die in den verschiedenen Ausführungsformen beschriebenen und in den Figuren gezeigten Ausgestaltungen und Varianten der Erfindung beliebig untereinander kombinierbar sind. Dabei sind einzelne oder mehrere Merkmale beliebig gegeneinander austauschbar. Diese Merkmalskombinationen sind ebenso mit offenbart.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die nur in der Beschreibung offenbart wurden oder auch Einzelmerkmale aus Ansprüchen, die eine Mehrzahl von Merkmalen umfassen, können jederzeit als von erfindungswesentlicher Bedeutung zur Abgrenzung vom Stande der Technik in den oder die unabhängigen Anspruch/Ansprüche übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

## Patentansprüche

1. Versorgungsvorrichtung für Inkontinenz bei Frauen umfassend
- ein Urinsammelelement (2) mit einer Urinableitung (21),
- eine Unterhose (3) mit einer Durchführung (31) für die Urinableitung (21),
- und ein Fixierelement (4),
wobei die Urinableitung (21) des Urinsammelelementes (2) durch die Durchführung (31) in der Unterhose (3) geführt ist und das Fixierelement (4) auf der, dem Urinsammelelement (2) abgewandten Seite der Unterhose (3) an der Urinableitung (21) befestigt ist und das Urinsammelelement (2) an der Unterhose (3) ortsfest und verdrehsicher festhält.

2. Versorgungsvorrichtung nach Anspruch 1, **dadurch gekennzeichnet, dass** das Fixierelement (4) form- und/oder kraftschlüssig mit der Urinableitung (31) verbunden ist und die Unterhose (3) zwischen dem Urinsammelelement (2) und dem Fixierelement (4) eingeklemmt ist.

3. Versorgungsvorrichtung nach einem der vorhergehenden Ansprüche, **dadurch gekennzeichnet, dass** das Urinsammelelement (2) zumindest eine Positionsführung (22) aufweist und die Unterhose (3) und/oder das Fixierelement (4) eine Führungsaufnahme (5) aufweist/aufweisen und die Positionsführung (22) und die Führungsaufnahme (5) formschlüssig miteinander in Eingriff bringbar sind um ein Verdrehen des Urinsammelelementes (2) zur Unterhose und/oder zum Fixierelement (4) zu verhindern.

4. Versorgungsvorrichtung nach einem der vorhergehenden Ansprüche, **dadurch gekennzeichnet, dass** das Urinsammelelement (2) eine Auffangschale (23) aufweist, welche schalenförmig und elastisch ausgebildet ist und die Auffangschale (23) zwischen den Schamlippen der Trägerin einbringbar ist, wobei eine Auffangöffnung (24) der Auffangschale (23) nach oben orientiert ist und den Harnröhrenausgang der Trägerin umgibt.

5. Versorgungsvorrichtung nach Anspruch 4, **dadurch gekennzeichnet, dass** das Urinsammelelement (2) zusätzlich zur Auffangschale (23) eine Fixiereinlage (25) aufweist, welche lösbar fest mit der Auffangschale (23) verbindbar ist, wobei die Fixiereinlage (25) als gewölbte Platte ausgeführt ist und eine, in der Draufsicht betrachtet, im Wesentlichen dreieckige Form aufweist.

6. Versorgungsvorrichtung nach Anspruch 5, **dadurch gekennzeichnet, dass** die Fixiereinlage (25) zwischen den Oberschenkeln im Schritt der Trägerin einbringbar ist wobei ein breiterer Bereich der Fixiereinlage (25) im unteren Bauchbereich der Trägerin anbringbar und ein schmalerer Bereich der Fixiereinlage (25) im Anusbereich der Trägerin anbringbar ist.

7. Versorgungsvorrichtung nach einem der Ansprüche 5 bis 6, **dadurch gekennzeichnet, dass** die Fixiereinlage (25) aus einem steiferen Material als die Auffangschale (23) besteht.

8. Versorgungsvorrichtung nach einem der vorhergehenden Ansprüche, **dadurch gekennzeichnet, dass** das Urinsammelelement (2) einen Vaginalanker (26) aufweist, der in die Vagina der Trägerin einführbar ist um das Urinsammelelement (2) zusätzlich zu fixieren, wobei der Vaginalanker (26) im Wesentlichen walzenförmig ausgebildet ist.

9. Versorgungsvorrichtung nach Anspruch 8, **dadurch gekennzeichnet, dass** der Vaginalanker (26) in einem Winkel α zur von der Auffangöffnung (24) aufgespannten Ebene angeordnet ist.

10. Versorgungsvorrichtung nach einem der vorhergehenden Ansprüche, **dadurch gekennzeichnet, dass** das Urinsammelelement (2) aus Gummi, Latex, PVC,... besteht.

11. Versorgungsvorrichtung nach einem Ansprüche 1 bis 4, **dadurch gekennzeichnet, dass** das Urinsammelelement (2) die Form eines Kajaks aufweist, welches bereichsweise zwischen den Schamlippen der Trägerin anbringbar ist.

12. Versorgungsvorrichtung nach Anspruch 11, **dadurch gekennzeichnet, dass** das kajakförmig ausgeführte Urinsammelelement (2) zumindest zwei Halteflügel (27) aufweist, welche seitlich am Urinsammelelement angeordnet sind und welche nicht innerhalb der Schamlippen der Trägerin einbringbar sind und welche im Bereich zwischen den Schamlippen und den Innenseiten der Oberschenkel der Trägerin einbringbar sind.

13. Versorgungsvorrichtung nach einem der Ansprüche 11 bis 12, **dadurch gekennzeichnet, dass** das kajakförmig ausgeführte Urinsammelelement (2) eine Dichtfläche aufweist, welche am oberen Rand des Urinsammelelementes (2) angeordnet ist und bei verwendeter Versorgungsvorrichtung außerhalb der Schamlippen der Trägerin anliegt.

14. Set umfassend
- eine Versorgungsvorrichtung nach einem der vorhergehenden Ansprüche
- und Mittel zur positionsgenauen Anbringung der Durchführung (31) in der Unterhose (3).

15. Set nach dem vorhergehenden Anspruch, **dadurch gekennzeichnet, dass** die Mittel zur positionsgenauen Anbringung der Durchführung (31) ein Markierungselement, insbesondere einen textilfesten Filzstift und ein Schneidelement, insbesondere einen Schneidstempel umfassen.
